# EUROPEAN PATENT SPECIFICATION

(11) **EP 1 240 317 B1**
(45) Date of publication and mention of the grant of the patent: **28.02.2007**
(21) Application number: 00985646.9
(22) Date of filing: 20.12.2000
(51) Int. Cl.: C12N 15/00, C07K 14/47

(54) **NUCLEIC ACID VACCINATION**
DNS IMPFUNG
VACCINATION PAR UN ACIDE NUCLEIQUE

(30) Priority: 22.12.1999 GB 9930359
(43) Date of publication of application: 18.09.2002
(73) Proprietor: GLAXO GROUP LIMITED, Greenford, Middlesex UB6 ONN (GB)
(72) Inventor: CROWE, James Scott, Glaxo Wellcome plc, Hertfordshire SG1 2NY (GB); ELLIS, Jonathan Henry, Glaxo Wellcome plc, Stevenage, Hertfordshire SG1 2NY (GB)
(74) Representative: Giddings, Peter John
(86) International application number: PCT/GB2000/004906
(87) International publication number: WO 2001/046228

(56) References cited:
- EP-A- 0 659 768
- WO-A-97/11715
- WO-A-97/34921
- DE-A- 19 516 673
- US-A- 5 744 144
- X LIU ET AL: "Structurally defined synthetic cancer vaccines: analysis of structure, glycosylation and recognition of cancer associated mucin, MUC-1 derived peptides" GLYCOCONJUGATE JOURNAL,GB,CHAPMAN & HALL, vol. 12, no. 5, October 1995 (1995-10), pages 607-617, XP002112485 ISSN: 0282-0080
- U KARSTEN ET AL: "Enhanced binding of antibodies to the DTR motif of MUC1 tandem repeat peptide is mediated by site-specific glycosylation" CANCER RESEARCH,US,AMERICAN ASSOCIATION FOR CANCER RESEARCH, BALTIMORE, MD, vol. 58, no. 12, 15 June 1998 (1998-06-15), pages 2541-2549, XP002112486 ISSN: 0008-5472
- PECHER GABRIELE ET AL: "Induction of cellular immunity in chimpanzees to human tumor-associated antigen mucin by vaccination with MUC-1 cDNA-transfected Epstein-Barr virus-immortalized autologous B cells." PROCEEDINGS OF THE NATIONAL ACADEMY OF SCIENCES OF THE UNITED STATES, vol. 93, no. 4, 1996, pages 1699-1704, XP002167404 1996 ISSN: 0027-8424

## Description

The present invention relates to nucleic acid vaccine constructs comprising isolated polynucleotides encoding polypeptides which are glycosylation variants of the MUC1 protein, vaccine compositions comprising the constructs and use of the constructs or compositions for vaccinating a mammal.

### Background of the Invention

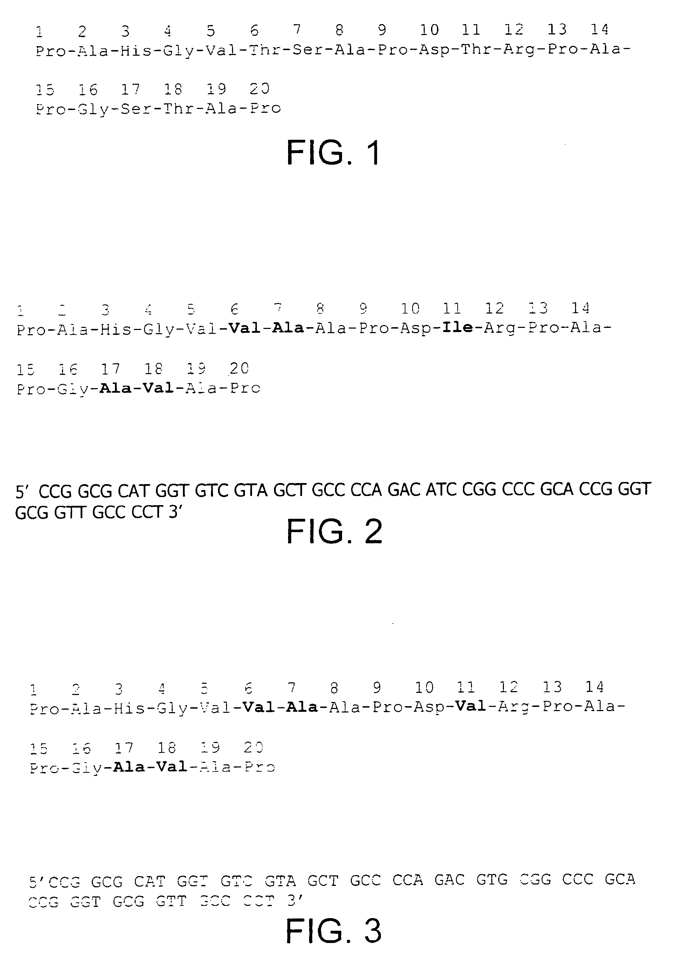

The epithelial cell mucin MUC1 (also known as episialin or polymorphic epithelial mucin, PEM) is a large molecular-weight glycoprotein expressed on many epithelial cells. The protein consists of a cytoplasmic tail, a transmembrane domain and a variable number of tandem repeats of a 20 amino acid motif (herein termed the VNTR monomer, it may also be known as the VNTR epitope, or the VNTR repeat) containing a high proportion of proline, serine and threonine residues. The number of repeats is variable due to genetic polymorphism at the MUC1 locus, and most frequently lies within the range 30-100 (Swallow et al, 1987, Nature **328**:82-84). In normal ductal epithelia, the MUC1 protein is found only on the apical surface of the cell, exposed to the duct lumen (Graham et al, 1996, Cancer Immunol Immunother **42**:71-80; Barratt-Boyes et al, 1996, Cancer Immunol Immunother **43:**142-151). One of the most striking features of the MUC1 molecule is its extensive O-linked glycosylation. There are five O-linked glycosylation sites available within each MUC1 VNTR monomer. According to the numbering system in Figure 1, these are Thr-6, Ser-7, Thr-11, Ser-17 and Thr-18.

In malignant carcinomas arising by neoplastic transformation of these epithelial cells, several changes affect the expression of MUC1. The polarised expression of the protein is lost, and it is found spread over the whole surface of the transformed cell. The total amount of MUC1 is also increased, often by 10-fold or more (Strous & Dekker, 1992, Crit Rev Biochem Mol Biol **27**:57-92). Most significantly, the quantity and quality of the O-linked carbohydrate chains changes markedly. Fewer serine and threonine residues are glycosylated. Those carbohydrate chains that are found are abnormally shortened, creating the tumour-associated carbohydrate antigen STn (Lloyd et al, 1996, J Biol Chem, **271**:33325-33334). As a result of these glycosylation changes, various epitopes on the peptide chain of MUC1 which were previously screened by the carbohydrate chains become accessible. One epitope which becomes accessible in this way is formed by the sequence APDTR (Ala 8 - Arg 12 in Figure 1) present in each 20 amino acid VNTR monomer (Burchell et al, 1989, Int J Cancer **44**:691-696).

It is apparent that these changes in MUC1 mean that a vaccine that can activate the immune system against the form of MUC1 expressed on tumours may be effective against epithelial cell tumours, and indeed other cell types where MUC1 is found, such as T cell lymphocytes. One of the main effector mechanisms used by the immune system to kill cells expressing abnormal proteins is a cytotoxic T lymphocyte immune response (CTL's) and this response is desirable in a vaccine to treat tumours, as well as an antibody response. A good vaccine will activate all arms of the immune response. However, current carbohydrate and peptide vaccines such as Theratope or BLP25 (Biomira Inc, Edmonton, Canada) preferentially activate one arm of the immune response - a humoral and cellular response respectively, and better vaccine designs are desirable to generate a more balanced response.

Nucleic acid vaccines provide a number of advantages over conventional protein vaccination, in that they are cheap, and easy to produce in large quantity. Even at small doses they have been reported to induce strong immune responses, and can induce a cytotoxic T lymphocyte immune response as well as an antibody response. However, there is a technical barrier preventing the development of effective nucleic acid vaccines against MUC1.

In nucleic acid vaccination, a nucleic acid molecule encoding the antigen of choice is introduced into normal cells of the host. If a nucleic acid vaccine encoding a MUC1 polypeptide is delivered by partide-mediated gene transfer (US patent 5371015), the most frequently transfected cells are likely to be keratinocytes or skin Langerhans cells. Similarly, if the nucleic acid is delivered by intramuscular injection, skeletal muscle cells and bone-marrow derived antigen presenting cells are the major target cell types. Each of these cells contains a physiological balance of glycosylation enzymes, and so will glycosylate the MUC1 gene product encoded by the vaccine in such a way as to more resemble MUC1 on normal epithelia, rather than the aberrantly glycosylated form found on transformed cells. Indeed, almost any cell which is transfected with a polynucleotide encoding MUC1 will glycosylate the gene product encoded by the vaccine. As a result, whilst it may be possible to see some results, the vaccine will fail to optimally stimulate immunity against the tumour cells.

Accordingly, the present invention provides nucleic acid vaccination constructs encoding a polypeptide comprising the VNTR monomer or repeats or fragments thereof, which have been manipulated such that the encoded sequence cannot be fully glycosylated by the normal cellular mechanisms. Surprisingly, the present inventors have managed to achieve this in one aspect by engineering novel amino acid substitutions in the encoded polypeptide. The inventors have produced nucleic acid vaccination constructs comprising polynucleotides which encode polypeptides that retain the conformation of the MUC1 epitopes, an essential requirement for continued immunogenicity of the altered polypeptides, and which have reduced glycosylation, hence resembling more closely the form of MUC1 expressed on turnouts.

### Summary of the Invention

According to one embodiment of the present invention there is provided a nucleic acid vaccine construct comprising an altered MUC-1 VNTR monomer in which the construct comprises the sequence shown in Figure 2 or Figure 3 and in which the encoded polypeptide, when expressed, can bind the antibody SM3.

In one aspect, the polypeptide encoded by the nucleic acid vaccine construct consists of one copy of the VNTR monomer of MUC1. In another aspect the polypeptide encoded by the nucleic acid vaccine construct consists of a fragment of the VNTR monomer of MUC1.

In a further embodiment of the invention is provided a vaccine composition comprising a nucleic acid vaccination construct as defined herein in combination with a pharmaceutically acceptable carrier.

In another embodiment of the invention is provided a nucleic acid vaccination construct or a vaccine composition as defined herein for use in vaccination of a mammal against tumours.

In yet another embodiment of the invention is provided use of a nucleic acid vaccination construct or a vaccine composition as defined herein for the manufacture of a medicament for use in vaccination of a mammal against tumours.

### Brief Description of the Figures

The invention will further be described by way of example, and with reference to the following figures:
Figure 1. This shows the Wild Type amino acid sequence of one MUC1 VNTR monomer element (termed here VNTR).
Figure 2. This shows the sequence of one polypeptide termed Mut5l encoded by a preferred isolated polynucleotide comprised on a nucleic acid vaccination construct of the invention. Mutations compared to the Wild Type sequence are shown in bold. Also included in figure 2 is a representative nucleotide sequence that encodes for this polypeptide.
Figure 3. This shows the sequence of one polypeptide termed Mut5V encoded by a preferred isolated polynucleotide comprised on a nucleic acid vaccination construct of the invention. Mutations compared to the Wild Type sequence are shown in bold. Also included in figure 3 is a representative nucleotide sequence that encodes for this polypeptide.

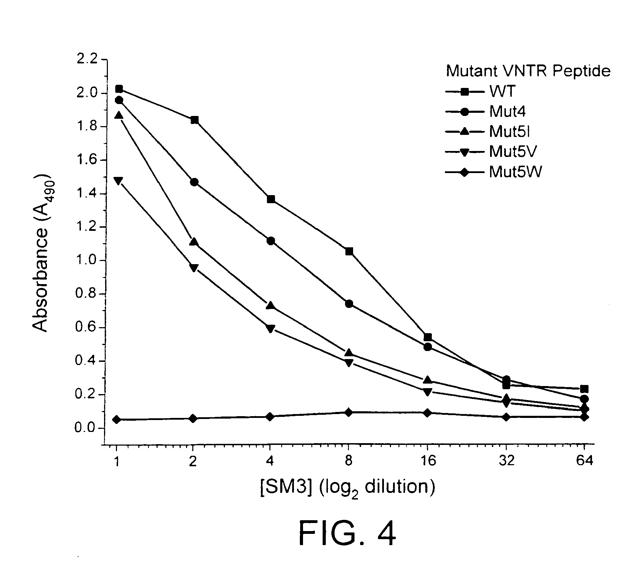

Figure 4. This shows antibody binding to wild type MUC1 polypeptides, and the mutated polypeptides shown in Table 1, across a range of antibody concentrations. These results demonstrate the ability of the antibody SM3 (which recognises the APDTRP motif at residues 8-13) to recognise the unglycosylated polypeptides. Mut5P, Mut 5A and Mut5N demonstrated substantially the same results as those shown for Mut5W.

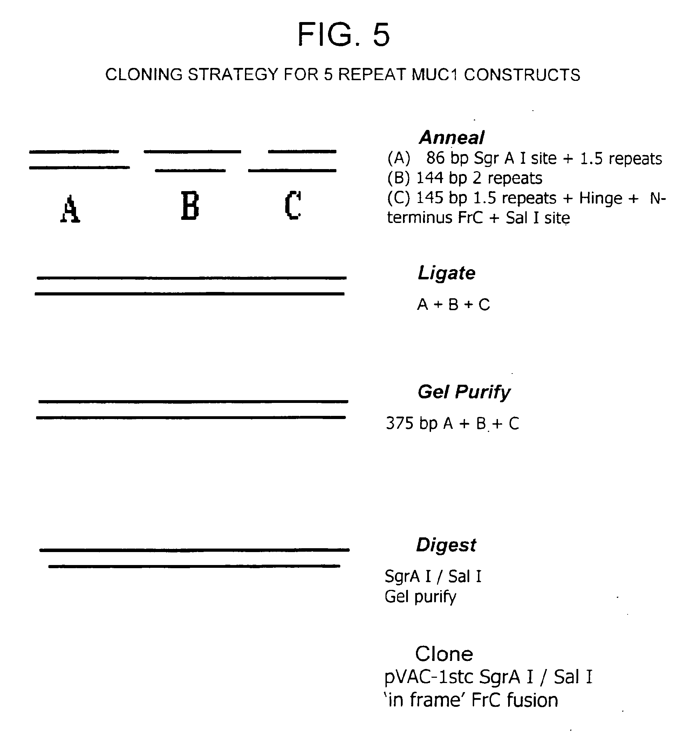

Figure 5. This demonstrates the cloning strategy used to produce the nucleic acid vaccine constructs containing mutant forms of Muc 1.

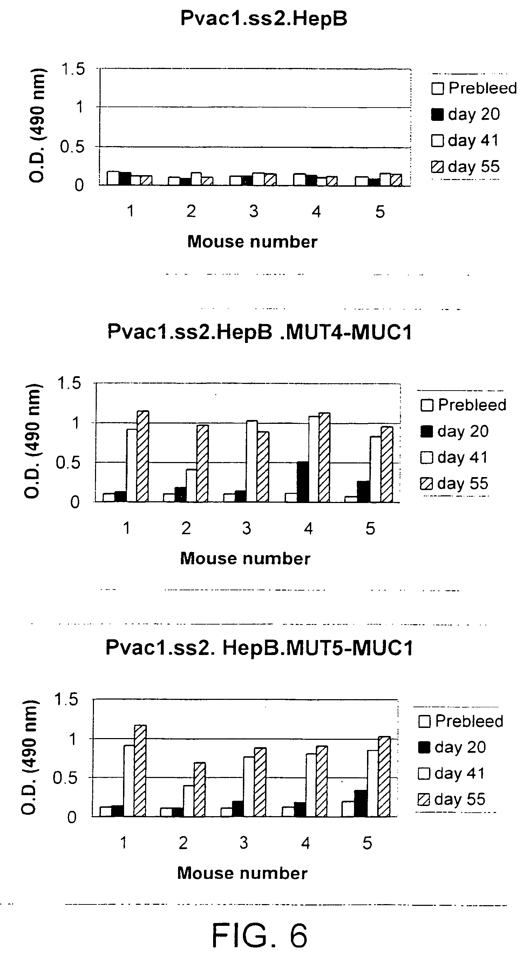

Figure 6. Antibody sera were taken from mice immunised with nucleic acid vaccine constructs encoding mutant forms of MUC 1. This figure shows the binding of that sera to a the wild type form of MUC1 in the form of a synthetic peptide. This shows that the nucleic acid vaccine constructs of the invention can raise antibodies *in vivo* that recognise the unmutated form of MUC1.

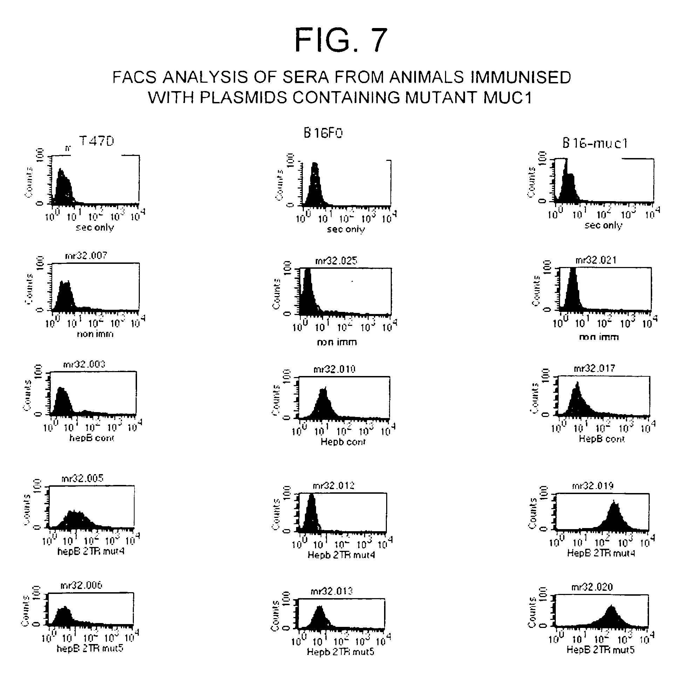

Figure 7. This shows a FACS analysis of the binding of antibody sera from mice immunised with nucleic acid vaccine constructs encoding mutant forms of MUC 1 to human breast cancer cells.

These results demonstrate that the nucleic acid vaccine constructs of the invention can raise antibodies *in vivo* that recognise the form of MUC1 expressed on tumours.

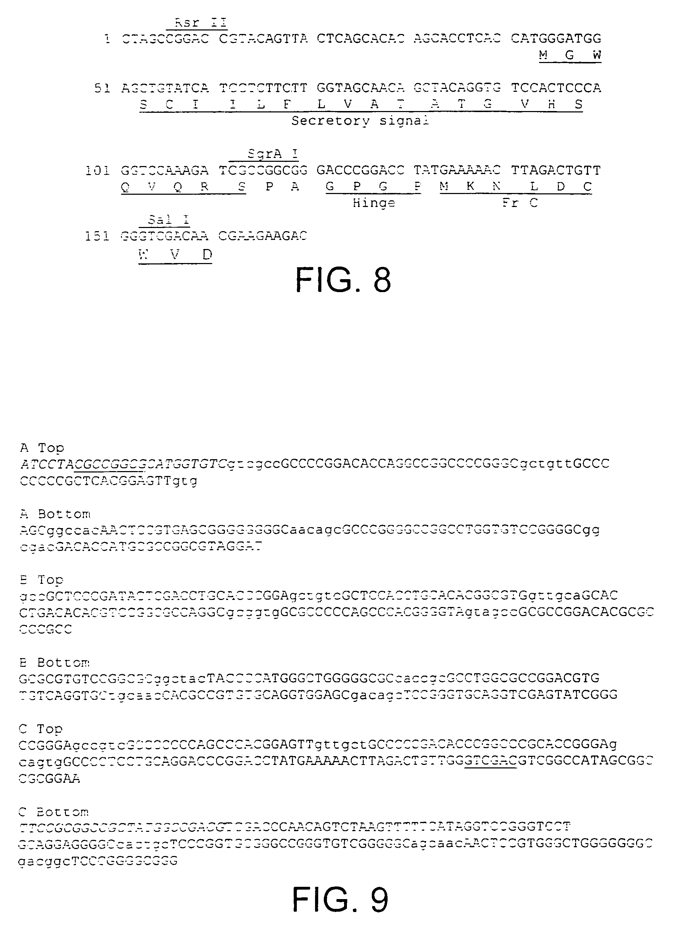

Figure 8. This shows the sequence of the N-terminal portion of pVAC1-stc, as described in example 3.
Figure 9. This shows the oligonucleotides used to construct MUT4 mutant MUC1 plasmids, as described in example 3. All sequences are written 5' to 3'. Underlining indicates restriction sites, italics indicate the sequences used as short PCR primers. Lower case letters indicate the departures from the wild-type sequence necessary to encode the desired mutations. The notation 'A Top', 'A Bottom' etc refers to the strategy shown in Figure 5.

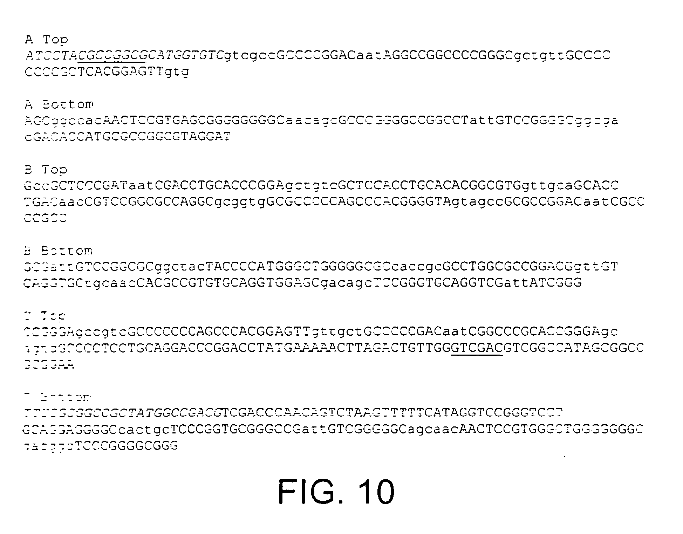

Figure 10. This shows the oligonucleotides used to construct MUT5N mutant MUC1 plasmids, as described in example 3. All sequences are written 5' to 3'. Underlining indicates restriction sites, italics indicate the sequences used as short PCR primers. Lower case letters indicate the departures from the wild-type sequence necessary to encode the desired mutations. The notation 'A Top', 'A Bottom' etc refers to the strategy shown in Figure 5.

### Detailed Description of the Invention

Throughout this specification and the appended claims, unless the context requires otherwise, the words "comprise" and "include" or variations such as "comprising", "comprises", "including", "includes" etc., are to be construed inclusively, that is, use of these words will imply the possible inclusion of integers or elements not specifically recited.

As described herein, the present invention relates to nucleic acid vaccine constructs comprising isolated polynucleotides, which constructs are useful in vaccination against tumours. In the context of this invention the term "isolated" is intended to convey that the polynucleotide is not in its native state, insofar as it has been purified at least to some extent or has been synthetically produced, for example by recombinant methods, or mechanical synthesis. The term "isolated" therefore includes the possibility of the polynucleotides being in combination with other biological or non-biological material, such as cells, suspensions of cells or cell fragments, proteins, peptides, expression vectors, organic or inorganic solvents, or other materials where appropriate, but excludes the situation where the polynucleotide is in a state as found in nature.

The polypeptides encoded by the isolated polynucleotides comprised in the nucleic acid vaccine constructs of the present invention have been termed "encoded polypeptides" throughout the specification and the claims. Said encoded polypeptides comprise at least five consecutive amino acid residues from the VNTR monomer of MUC1 wherein one or more of said amino acids is a glycosylation site, and glycosylation has been prevented or altered on at least one of said glycosylation sites. Preferably, said encoded polypeptides comprise at least 10, for example 13, 15, 16, 17, 18, or 19 amino acids from the VNTR monomer of MUC1. Particularly preferably, said encoded polypeptides comprise at least 20 amino acids from the VNTR monomer of MUC1. The consecutive amino acids may be entirely from one VNTR monomer, or may span across monomers, for example three of said consecutive amino acids may be from the N terminal end of one VNTR monomer, and the next two amino acids may be from the C terminal end of a second VNTR monomer.

The term "at least 5 consecutive amino acids" includes the instance where one or more of said amino acids has been altered from the wild type by an amino acid mutation. So, for example, an encoded polypeptide which has a sequence containing three amino acids according to the wild type sequence, then an amino acid substitution for the next wild type amino acid, then a further three amino acids according to the wild type sequence is considered to have at least five consecutive amino acids from the MUC1 VNTR monomer.

In one embodiment, the isolated polynucleotide comprised on the nucleic acid vaccine constructs of the invention is such that the encoded polypeptide consists of between one and ten copies of the VNTR monomer. In another embodiment the isolated polynucleotide comprised on the nucleic acid vaccine constructs of the invention is such that the encoded polypeptide consists of 10 or more copies of the VNTR monomer, for example 20, 30, 50, 60, 75, 90 or 100 or more copies of the VNTR monomer. In a further embodiment, the isolated polynucleotide comprised on the nucleic add vaccine constructs of the invention is such that the encoded polypeptide consists of a combination of repeats and fragments, for example one VNTR monomer and one fragment, between 1 and 10 repeats and 1 and 10 fragments, 10 or more repeats and 10 or more fragments, 10 or more repeats and 10 or less fragments or 10 or more fragments and 10 or less repeats. In a further embodiment, the isolated polynucleotide comprised on the nucleic acid vaccine constructs of the invention is such that the encoded polypeptide consists of a combination of fragments for example a number of copies of one fragment, or a number of different fragments. In each case, where more than one fragment is present in the encoded polypeptide, the fragments may be identical or different.

Preferably, the alteration or prevention of glycosylation is achieved by mutation of the amino adds making up the glycosylation sites within the encoded polypeptides. The term "amino acid mutation" as used throughout the specification and the claims means that one or more amino acids in the encoded polypeptides differs from the wild type MUC1 VNTR monomer sequence as shown in figure 1. This change may be, for example, deletion, insertion or substitution of one or more amino acids.

These mutations are achieved by designing the sequence of the isolated polynucleotide comprised in the nucleic acid vaccine construct such that the encoded polypeptide has amino acid mutations at one or more of the glycosylation sites. Where the desired amino acid mutation is a substitution, the three nucleotides encoding that amino acid in the wild type sequence will be changed to the nucleotides encoding the desired amino acid. Where the desired amino acid mutation is a deletion, the three nucleotides encoding that amino acid in the wild type sequence will be deleted. Where the desired amino acid mutation is an insertion, the three nucleotides encoding the desired amino acid will be inserted into the wild type nucleotide sequence.

A person skilled in the art will readily be able to determine the sequence of the polynucleotide required by applying the genetic code to the desired encoded polypeptide. Once the required sequence has been determined, the nucleic acid vaccine construct comprising the isolated polynucleotide with the desired sequence can be produced as described in the examples. A skilled person will readily be able to adapt any parameters necessary, such as primers and PCR conditions. It will also be understood by a person skilled in the art that, due to the degeneracy of the genetic code, there is potentially more than one isolated polynucleotide sequence that can be used to produce a desired encoded polypeptide.

In one embodiment, the nucleic acid vaccine construct comprises a nucleotide sequence comprising the sequence shown in figure 2, or the sequence shown in figure 3.

In a preferred embodiment, the sequence of the isolated polynucleotide is such that the amino acid mutation is a substitution. In a more preferred embodiment is the sequence of the isolated polynucleotide is such that the amino acid mutation is the substitution of a serine or threonine residue with another amino acid lacking the hydroxyl group which forms the site of attachment of O-linked glycosylation. Preferably, sequence of the isolated polynucleotide is such that serine or threonine is replaced with valine, isoleucine, alanine, asparagine, phenylalanine or tryptophan. More preferably, the sequence of the isolated polynucleotide is such that threonine or serine is substituted with Valine or Isoleucine. Even more preferably, the sequence of the isolated polynucleotide is such that threonine is substituted with valine or isoleucine. In a particularly preferred embodiment, the sequence of the isolated polynucleotide is such that the encoded polypeptide has theronine 11 substituted by valine or isoleucine, and especially preferred sequences are shown in figures 2 or 3, with the amino acid substitutions highlighted. Preferably, the sequence of the isolated polynucleotide is such that the encoded polypeptides have been mutated such that glycosylation has been altered or prevented on at least 60%, for example 70%, 75%, 80%, 85%, 90% or 100% of the glycosylation sites present in the polypeptides.

The encoded polypeptides preferably retain immunological similarity to the form of MUC1 found on tumours. The term "immunological similarity" is intended to mean that despite the mutation of one or more amino acids, the conformation of one or more epitopes in the encoded polypeptide remains sufficiently unchanged so that an antibody that would recognise an epitope in the VNTR monomer of the form of MUC1 expressed on tumours would also recognise that epitope in the encoded polypeptides. The term "immunological similarity" encompasses not only the situation where all antibodies that recognise the form of MUC1 expressed on tumours also recognise the encoded polypeptides, but also any situation where at least one epitope remains sufficiently unchanged for at least one anti-MUC1 antibody to recognise the encoded polypeptides. It is particularly preferred that the antibodies raised by the encoded polypeptides can distinguish between the form of MUC1 expressed on tumour cells, and the form of MUC1 expressed on normal cells, for example by binding to the former but not the latter.

In one embodiment the encoded polypeptides, when expressed, are those that can be recognised by the anti-MUC1 antibody SM3.

The nucleic acid vaccination constructs of the invention may also comprise a further isolated polynucleotide encoding a heterologous polypeptide, preferably an immunogenic polypeptide. This isolated polynucleotide is situated on the nucleic acid vaccination construct such that, when expressed, the heterologous polypeptide is linked or fused to the expressed encoded polypeptide. Preferably, the isolated polynucleotide encoding the heterologous polypeptide is contiguous to the isolated polynucleotide encoding the encoded polypeptides.

In one embodiment the encoded heterologous polypeptides may act as carriers to target the encoded polypeptides, for example the heterologous polypeptide may bind to a receptor on a target cell. In another embodiment, the heterologous polypeptide may act as an immunogen to enhance the immune response elicited by the encoded polypeptides Suitable heterologous polypeptides include keyhole limpet haemacyanin, ovalbumin, hepatitis B virus surface protein, hepatitis B virus core protein, tetanus toxin, glutathione S transferase or pigeon cytochrome C. In a preferred embodiment, the heterologous polypeptide is tetanus toxin or hepatitis B virus core protein. The nucleotide sequences encoding these polypeptides are readily available to a person skilled in the art.

Additionally, the nucleic acid vaccination construct will comprise appropriate initiators, promoters, enhancers and other elements, such as for example polyadenylation signals which may be necessary, and which are positioned in the correct orientation, in order to allow for protein expression within a mammalian cell.

The promoter may be a eukaryotic promoter for example a CD68 promoter, Gal1, Gal10, or NMT1 promoter, a prokaryotic promoter for example Tac, Trc, or Lac, or a viral promoter, for example the cytomegalovirus promoter, the SV40 promoter, the polyhedrin promoter, the P10 promoter, or the respiratory syncytial virus LTR promoter. Preferably the promoter is a viral promoter. Particularly preferred is when the promoter is the cytomegalovirus immediate early promoter.

The transcriptional regulatory elements may comprise enhancers, for example the hepatitis B surface antigen 3'untranslated region, the CMV enhancer; introns, for example the CD68 intron, or the CMV intron A, or regulatory regions, for example the CMV 5' untranslated region.

The nucleic acid vaccine construct backbone may be RNA or DNA, for example plasmid DNA, viral DNA, bacterial DNA, bacterial artificial chromosome DNA, yeast artificial chromosome DNA, synthetic DNA It is also possible for the nucleic acid vaccine construct to be artificial nucleic acid, for example phosphorothioate RNA or DNA. Preferably the construct is DNA, particularly preferred is when it is plasmid DNA.

The isolated polynucleotide comprised on the nucleic acid vaccine construct which encodes an encoded polypeptide may be RNA, for example mRNA or may be DNA, for example genomic DNA, cDNA or synthetic DNA. Preferably the polynucleotide is DNA, particularly preferably it is cDNA. The polynucleotide is preferably operably linked to the promoter on the nucleic acid vaccine construct such that when the construct is inserted into a mammalian cell, the polynucleotide is expressed to produce a encoded polypeptide.

The nucleic acid vaccine constructs of the present invention produce the encoded polypeptides when expressed in a mammalian cell. Said mammalian cell may be *in vitro,* in a cell line in culture in a laboratory, for example, HEK293T, CHO, HeLa or COS cells. In this case the expressed polypeptides may be harvested and themselves used for vaccination. More preferably, the mammalian cell is *in vivo,* and the nucleic acid vaccine construct is administered directly to a mammal, for example a mouse, dog, cat, rabbit, pig, cow, horse or rat or, particularly preferably, a human.

The present invention also includes vaccine compositions, which comprise a therapeutically effective amount of nucleic acid vaccine construct of the invention, preferably in combination with a pharmaceutically acceptable carrier such as phosphate buffered saline (PBS), saline, dextrose, water, glycerol, ethanol, or combinations thereof. The vaccine composition may alternatively comprise a therapeutically effective amount of a nucleic acid vaccine construct of the invention, formulated onto gold beads. Alternatively, the composition may comprise the nucleic acid vaccine constructs formulated with liposomes. The nucleic acid vaccine construct will be such that when administered to a mammal, the polypeptide is expressed within that mammal. The expressed or administered polypeptide will then generate an immune response, which preferably involves both humoral and cellular immunity.

The present invention also includes nucleic acid vaccination constructs or vaccine compositions as described herein for use in vaccination of a mammal against tumours both for prophylaxis and therapy. Preferably the mammal is a human. The vaccination may be directed against a tumour of any cell type which expresses MUC1 and so the tumour may be responsive to antibodies which recognise the MUC1 VNTR monomer. In one embodiment, the tumours are of T lymphocytes. Preferably the vaccination is directed against tumours of epithelial cells, more preferably the vaccination is directed against breast cancer or non-small cell lung cancer.

The nucleic acid vaccine constructs and vaccine compositions including them may be administered in a variety of manners for example via the oral, nasal, pulmonary, intramuscular, subcutaneous or intradermal routes. They may be administered to an individual as an injectable composition, for example as a sterile aqueous dispersion, preferably isotonic. One particularly preferred technique involves particle bombardment (which is also known as 'gene gun' technology and is described in US Patent No. 5371015). Here inert particles (such as gold beads) are coated with a nucleic acid, and are accelerated at speeds sufficient to enable them to penetrate a surface of a recipient (e.g. skin), for example by means of discharge under high pressure from a projecting device. (Particles coated with nucleic acid vaccine constructs of the invention are within the scope of the present invention, as are devices loaded with such particles.) Other methods of administering the nucleic acid vaccine constructs or compositions containing said constructs directly to a recipient include ultrasound, electrical stimulation, electroporation and microseeding which is described in US-5,697,901.

A nucleic acid vaccine construct of the present invention may also be administered by means of specialised delivery vectors useful in gene therapy. Gene therapy approaches are discussed for example by Verme *et al,* Nature 1997, **389**:239-242. Both viral and non-viral systems can be used. Viral based systems include retroviral, lentiviral, adenoviral, adeno-associated viral, herpes viral and vaccinia-viral based systems. Non-viral based systems include direct administration of nucleic acids and liposome-based systems. For example, the vectors may be encapsulated by liposomes or within polylactide co-glycolide (PLG) particles.

A nucleic acid vaccine construct of the present invention may also be administered by means of transformed cells. Such cells include cells harvested from a subject. The nucleic acid vaccine construct can be introduced into such cells *in vitro* and the transformed cells can later be returned to the subject. The nucleic acid vaccine construct of the invention may integrate into nucleic acid already present in a cell by homologous recombination events. A transformed cell may, if desired, be grown up *in vitro* and one or more of the resultant cells may be used in the present invention. Cells can be provided at an appropriate site in a patient by known surgical or microsurgical techniques (e.g. grafting, micro-injection, etc.)

The amount of nucleic acid vaccine constructs or composition containing them which is delivered will vary significantly, depending upon the species and weight of mammal being immunised, the nature of the disease state being treated/protected against, the vaccination protocol adopted (i.e. single administration versus repeated doses), the route of administration and the potency and dose of the adjuvant compound chosen. Based upon these variables, a medical or veterinary practitioner will readily be able to determine the appropriate dosage level but it may be, for example, 0.5-5µg/kg of the nucleic acid vaccine constructs or composition containing them. In particular, the dose will vary depending on the route of administration. For example, when using intradermal administration on gold beads, the total dosage will preferably between 1µg - 10ng, particularly preferably, the total dosage will be between 10µg and 1 ng. When the nucleic acid vaccine construct is administered directly, the total dosage is generally higher, for example between 50µg and 1 or more milligram. The above dosages are exemplary of the average case. There can, of course, be individual instances where higher or lower dosage ranges are merited, and such are within the scope of this invention.

It is possible for the nucleic acid vaccine construct comprising the polynucleotide sequence encoding the antigenic peptide or the composition comprising the nucleic acid vaccine construct, to be administered on a once off basis or to be administered repeatedly, for example, between 1 and 7 times, preferably between 1 and 4 times, at intervals between about 1 day and about 18 months, preferably one month. This may be optionally followed by dosing at regular intervals of between 1 and 12 months for a period up to the remainder of the patients life. Once again, however, this treatment regime will be significantly varied depending upon the size and species of animal concerned, the amount of nucleic acid vaccine construct or composition administered, the route of administration, the potency and dose of any adjuvant compounds used and other factors which would be apparent to a skilled veterinary or medical practitioner.

The present invention will now be described, by way of example, in the following experimental section.

### Examples

Throughout the following examples of the invention, use is made of various widely known and practised techniques in molecular and cellular biology. Practical details of these may be found in a number of textbooks including Sambrook et al, 1989, Molecular Cloning, Cold Spring Harbor Press.

Numbering of residues within the MUC1 VNTR is according to the scheme shown in Figure 1.

### Example 1: Antibody binding capacity of variants of MUC1 lacking O-linked glycosylation sites.

Synthetic peptides were designed containing mutations at the glycosylation sites. Particular attention was focused on substitutions at the threonine residue in this sequence (T11). Table 1 below indicates the peptides that were designed and their mutated glycosylation sites. Peptides containing two identical copies of the designed VNTR were synthesised at Genemed Synthesis Inc (San Francisco, CA). These peptides were then screened for their ability to bind to a number of anti-MUC1 monoclonal antibodies in an ELISA.

**Table 1**

| Peptide | Sequence | Mutated glycosylation sites |
|---|---|---|
| WT | Ac-(PAHGVTSAPDTRPAPGSTAP)₂-NH₂ | None |
| Mut4 | Ac-(PAHGVVAAPDTRPAPGAVAP)₂-NH₂ | T6, S7, S17, T18 |
| Mut5A | Ac- (PAHGWAAPDARPAPGAVAP)₂-NH₂ | T6, S7, T11, S17, T18 |
| Mut5I | Ac- (PAHGVVAAPDIRPAPGAVAP)₂-NH₂ | T6, S7, T11, S17, T18 |
| Mut5N | Ac- (PAHGWAAPDNRPAPGAVAP) ₂-NH₂ | T6, S7, T11, S17, T18 |
| Mut5P | Ac- (PAHGVVAAPDPRPAPGAVAP) ₂-NH₂ | T6, S7, T11, S17, T18 |
| Mut5V | Ac- (PAHGWAAPDVRPAPGAVAP) ₂-NH₂ | T6, S7, T11, S17, T18 |
| Mut5W | Ac- (PAHGWAAPDWRPAPGAVAP) ₂-NH₂ | T6, S7, T11, S17, T18 |

Peptides were prepared as aqueous solutions in 50 mM sodium bicarbonate buffer pH9.6 and coated onto Nunc Maxisorp plates at 4°C overnight at a range of concentrations between 50 µg/ml and 25 ng/ml. After extensive washing with TBS-Tween (Tris-buffered saline, pH7.4 containing 0.05% Tween20), the plates were blocked with blocking solution (3% w/v bovine serum albumin in TBS-Tween) for 2 hours at room temperature. Anti-MUC1 antibodies HMFG1 (Novocastra, Newcastle, UK), HMFG2 (Novocastra), SM3 (Girling et al, 1989, Int J Cancer **43:**1072-1076) and ATR1 (Bynum et al, 1995, Hybridoma **14:**587-591) were diluted in blocking solution, added to the plate and incubated at room temperature for one hour. After washing, bound antibody was labelled by incubation with HRP-conjugated anti-mouse IgG (P260, Dako, Denmark) at 1/2000 dilution in blocking buffer. The plate was washed again and bound conjugate detected using Fast OPD colour reagents (Sigma, Poole, UK). The reaction was stopped by the addition of 3M sulphuric acid, and the OPD product quantitated by measuring the absorbance at 490 nm.

The results of this experiment are shown in Table 2. The ELISA signal for each antibody and peptide combination is ranked according to a semiquantitative scale, where +++ indicates strong binding, ++ indicates fair binding, + indicates weak binding, and - indicates no significant binding.

**Table 2**

| Peptide | Antibody SM3 | Antibody HMFG1 | Antibody HMFG2 | Antibody ATR1 |
|---|---|---|---|---|
| WT | +++ | +++ | +++ | +++ |
| Mut4 | +++ | ++ | +++ | +++ |
| Mut5A | - | ++ | - | +++ |
| Mut51 | +++ | ++ | +++ | ++ |
| Mut5N | - | - | - | +++ |
| Mut5P | - | + | - | + |
| Mut5V | +++ | - | +++ | +++ |
| Mut5W | - | - | - | ++ |

These data demonstrate that it is possible to alter all the O-linked glycosylation sites in the MUC1 VNTR monomer, in such a way as to prevent glycosylation, without perturbing the overall structure of the antigen, as evidenced by maintenance of binding of a number of anti-MUC1 antibodies. An especially surprising and useful finding is that the Mut51 and Mut5V versions of the VNTR maintain binding for the SM3 antibody. When used in immunohistochemical studies of clinical samples, SM3 shows strong selective staining for MUC1 expressed on tumour cells as opposed to MUC1 expressed on surrounding normal tissue (Girling et al, 1989). These findings clearly show that it is possible to derive variants of the VNTR which adopt a conformation similar to epitopes formed by the tumour form of MUC1 - a highly desirable property for a vaccine immunogen.

### Example 2: An antibody binding curve for the mutant polypeptides.

The polypeptides described in table 1 above were again used in this experiment. The methodology was essentially similar to Example 1 except that peptides were coated onto an ELISA plate at a fixed concentration of 3 ug/ml, and different concentrations of antibody SM3 were applied. The results are shown in Figure 4. Mut5P, Mut5A and Mut5N show antibody binding patterns essentially the same as those shown for Mut5W.

The data shown in figure 4 not only confirms that shown above, but illustrates that the mutation of Thr-11 to Ile and Val in Mut 5l and Mut5V does little to reduce the affinity of antibody SM3 for the peptide, indicating that non-glycosylatable mutants may indeed be very close mimics of the native sequence.

### Example 3: Nucleic Acid Vaccination with MUC1 mutants.

### Plasmid Preparation.

### Table 3: Primers used

| Name | Sequence 5'-3' |
|---|---|
| SSF | ATCCTACGGACCGTACAGTTACTCAGCACACACAGCACCTCAC |
| SSR | CCGCTCGAGCGCCGGCGATCTTGGACCTGGGAGTGGACACCTG |
| FrCF | ATCCTACGCCGGCGGGACCCGGACCTATGAAAAACTTAGACTGTT GGGTCGACAACGAAGAAGAC |
| FrCR | GGGCTCGAGTTAGTCGTTGGTCCAACCTTCATCGGTCGG |
| HBV1F | GATGTGGTCGACGACATTGACCCTTATAAAGAATTTGGAGC |
| HBV1R | GTAGAGCTCGAGCTAACATTGAGATTCCCGAGATTGAGATCTTCT GC |
| FrCS1 | GGCTGCGCGTTCCGAAAGTTTCTG |
| FrCS2 | CCGTGAGGACAACAACATCACTCT |
| FrCS3 | CTACTACCGACGTCTGTACAACGG |
| FrCS4 | GTTTCTTCATAGAGCTGATGATGG |
| FrCS5 | GAAGATACGGAACTTGTCGATGG |
| FrCS6 | GTAAGAAACGTACAGTTTGATGAAG |
| MUT4F | GATTGTGCTAGCCCAGCCCACGGAGTTGTTGCTGCC |
| MUT4R1 | TCGCGTATCTGGCGCTGCGACGACACCATGTGCTGGAGGGGCCAC TGCTCC |
| MUT4R2 | GATTGTGCTAGCAGGTGCTACGGCGCCGGGAGCCGGTCGCGTATC TGGCGCTGC |
| MUT5NF | GATTGTGCTAGCCCGGCGCATGGTGTCGTC |
| MUT5NR | GATTGTGCTAGCAGGGGCCACTGCTCC |

### Construction of pVAC1stc plasmid

The plasmid pVAC1 (Thomsen et al, *Immunology* 95:S1, OP106, 1998) is a eukaryotic expression vector optimised for DNA vaccination. It contains a CMV promoter operably linked to a multiple cloning site into which inserts encoding antigens may be placed. An insert containing a Kozak translation initiation signal, mammalian secretion signal and cloning sites was prepared by PCR using as template the plasmid pMNM1 (Ellis et al, *J. Immunology* 156: 2700-2709, 1996) and as primers SSF and SSR (Table 3). The PCR fragment was cleaved with Rsr II and Xhol to create a 120 bp insert which was cloned into the Rsr II and Xho I sites of pVAC1 to generate the plasmid pVac-1ss2. The DNA sequence was confirmed using the PCR amplification primers as sequencing primers. The second step was the introduction of an 'in frame' Tetanus toxin fragment C, (FrC), gene fused at the SgrA I site encoded by primer SSR, allowing for the addition of antigenic epitopes 'in frame' at this site. This was achieved by PCR amplification of the FrC gene from the plasmid pIC9Tet15, (Clare et al, *Methods in Molecular Biology* 103:193-208, 1998) using primers tagged with DNA encoding for a peptide hinge and restriction enzyme sites for SgrA I and Xhol (FrCF and FrCR respectively). The 1.3kb SgrA I / Xho I cleaved PCR fragment was cloned into the SgrA I and Xho I sites of pVac-1ss2 to generate the plasmid pVac-1stc. The DNA sequence was confirmed by fluorescent sequencing using the PCR amplification primers and the internal FrC derived sequences FrCS1 to FrCS6 (Table 3). The DNA sequence and amino acid sequence at the N terminus of the FrC fusion protein showing the epitope fusion sites is shown in figure 8.

### Construction of pVAC1ss2 MUC-1 2TR Fragment C plasmids

Sets of synthetic oligonucleotides were designed that upon annealing and subsequent cloning into the plasmid pVac-1stc would enable encoding a tandem arrays of five copies of the MUC1 VNTR epitope (PAHGVTSAPDTRPAPGSTAP). Two variants were designed: both were mutant versions in which four or five of the threonine and serine residues subject to glycosylation in each copy of the epitope had been replaced by non-modifiable amino acids: PAHGVVAAPDTRPAPGAVAP, four mutations, (MUT4) and PAHGVVAAPDNRPAPGAVAP, five mutations, (MUT5N). In all cases, the MUC1 sequences were to be expressed as a part of a secreted Fr C fusion protein. The oligonucleotides are listed in figures 9 and 10 (sites for the restriction enzymes SgrA 1 and Sal1 are underlined, lower case letters indicate mutations away from the wild-type sequence). The strategy used, (after treatment with T4 polynucleotide kinase to phosphorylate the 5' ends of the oligonucleotides), to produce the tandem repeats is shown in Figure 5. The strategy was used to generate a plasmid containing two tandem arrays of the Muc-1 MUT5N VNTR fused 'in frame' to FrC, (pVAC-1stcMUC-1 MUT5N), formed by homologous recombination and deletion from the five repeat sequence. To generate a similar plasmid containing the MUT4 VNTRs, a modification of the strategy described in Figure 5 was employed in that after the ligation stage the oligonucleotides were subject to PCR amplification. The primers used were the DNA sequences italicised in Figures 9 and 10, and PCR conditions were 25 cycles of 94°C for 45 seconds, 69-75°C for 45 seconds and 72°C for 1 minute. The PCR reactions were then processed as in Figure 5, but only the largest clean product, (generally around 150-200bp in size), was gel purified and cloned. Using this approach a plasmid containing one and a half repeats of the Muc-1 MUT4, (pVAC-1stcMUC-1MUT4), VNTR fused 'in frame' to FrC, was generated, formed by PCR-mediated strand jumping and deletion from the five repeat sequence.

An alternative way to generate the sequences described would be direct synthesis of oligonucleotides containing less copies of the VNTR repeats with a similar digestion and cloning strategy to that described above.

### Construction of pVAC1ss2 HepB Core plasmid

The plasmid pPA1 contains the gene for HBV core antigen (ADW serotype) with a unique Nhel site in the E1 loop region (Chambers et al, *J*. *Virol* 70:4045-4052, 1996). This cloning site allows insertion of a small fragment of DNA encoding a key epitope from a foreign protein. The E1 loop of the protein is exposed on the surface of expressed core antigen. The engineered HBV core insert was amplified by PCR using primers HBV1 F and HBV1R (Table 3), and cloned into pCR2.1 (Invitrogen) by the TA-overhang method to create pCR2.1-HepBE1. The construct was sequenced before use.

PCR was then used to amplify fragments encoding two tandem repeats of the MUC1 VNTR monomer from the pVAC-1stcMUC-1MUT4 and pVAC-1stcMUC-1MUT5N plasmids. The two tandem repeat MUT5N insert was prepared by PCR using pVAC-1stcMUC-1MUT5N as template and primers MUT5NF and MUT5NR (Table 3).

The plasmid pVAC-1stcMUC-1MUT4 only contained one complete tandem repeat of the MUC1 VNTR monomer. This plasmid was used as the template in a two stage PCR using a common forward primer (MUT4F) and two different reverse primers. In the first reaction, the reverse primer (MUT4R1) adds part of the additional sequence required to generate a second complete repeat of the MUT4 version of the MUC1 sequence. (ie: half a tandem repeat) to the existing tandem repeat. The resultant PCR product was then used as a template for a second round PCR using a second reverse primer (MUT4R2) to add on the residues required to complete the repeat, together with the required Nhel cloning site.

The MUT4 or MUT5 PCR products were digested with Nhel and cloned into vector pCR2.1 HepBE1 cut with Nhel. Resultant clones were orientated and sequence verified by fluorescent dideoxy sequencing. The full HepB core + MUC-1 TR cassette region was then excised using Sall and Xhol and cloned into pVAC1ss2 cut with Xhol Final constructs were all verified by full sequence analysis, creating pVAC1.ss2.MUT4.MUC1.HepB (encodes two copies of the MUT4 variant of the MUC1 VNTR monomer in the E1 loop of HBV core protein); and pVAC1.ss2.MUT5N.MUC1.HepB (encodes two copies of the MUT5N variant of the MUC1 VNTR monomer in the E1 loop of HBV core protein).

### DNA Vaccination with mutant MUC1 constructs

Plasmid DNA was precipitated onto 2 µm diameter gold beads using calcium chloride and spermidine. Loaded beads were coated onto Tefzel tubing as described (Eisenbraum et al, *DNA Cell Biology* 12:791-797, 1993; Pertmer et al, *J*. *Virol* 70:6119-6125, 1996). Particle bombardment was performed using the Accell gene delivery system (PCT WO 95/19799). For each plasmid, five female C56BI/6 mice were immunised with 3 administrations of plasmid on days 0, 21 and 42. Each administration consisted of two bombardments with DNA/gold, providing a total dose of approximately 2.5 µg of plasmid.

Serum samples were obtained from the animals by venepuncture on days - 1, 20, 41 and 55, and assayed for the presence of anti-MUC1 antibodies. ELISA was performed using Nunc Maxisorp plates coated overnight at 4°C with 3 µg/ml of wt MUC1 sequence (40 mer corresponding to 2 tandem repeat). After washing with TBS-Tween (Tris-buffered saline, pH 7.4 containing 0.05 % of Tween 20) the plates were blocked with 3 % BSA in TBS-Tween buffer for 2 h at room temperature. All sera were incubated at 1:100 dilution for 1 h at RT in TBS-Tween buffer. Antibody binding was detected using HRP-conjugated rabbit anti-mouse immunoglobulins (Dako, Denmark) at 1:2000 dilution in TBS-Tween buffer. Plates were washed again and bound conjugate detected using Fast OPD colour reagents (Sigma, Poole, UK). The reaction was stopped by the addition of 3M sulphuric acid, and the OPD product quantitated by measuring the absorbance at 490 nm.

The results of this analysis are shown in Figure 6. They demonstrate that glycosylation mutant sequences of the present invention may be used as vaccines to elicit immune responses capable of recognising the wild-type MUC1 sequence.

In order to demonstrate that antibodies evoked by these vaccines are capable of recognising tumour cells, samples of antisera from these mice were used to label various tumour cell lines, and the labelling visualised by flow cytometry.

Cells (T67-D, MCF-7, B16F0 and B16F0MUC1;1x10⁶) were washed in PBS buffer supplemented with 5% FCS and incubated for at 4°C for 15 min with mouse sera at 1:100 dilution. After washing, cells were incubated with the second antibody (Sheep anti-mouse IgG, Dako, Denmark, at 1:10 dilution) under the same conditions. Control cells were incubated with FACS buffer instead of the first step antibody prior to staining with the second step reagent. FACS analysis was performed using a FACScan (Becton Dikinson). One thousand cells per sample were simultaneously measured for FSC (forward angle light scatter) as SSC (integreated light scatter) as well as green (FL1) and red (FL3) fluorescences (expressed as logarithm of the integrated fluorescence light). Recordings were made only in propidium iodide-negative (viable) cells of the red fluorescence, excluding aggregates whose FCS were out of range. Data were expressed as histograms plotted as number of cells (Y-axis) versus fluorescence intensity (X-axis) for the different types of mouse sera bound to the surface of the tumour cells.

The results can be seen in figure 7. This figure shows that serum from mice immunised with the glycosylation mutant MUC1 constructs does indeed contain anti-MUC1 IgG capable of binding to both human and murine tumour cells expressing native cell-surface MUC1. T47D is the human tumour breast cell line. B16FO is a parental B16 cell line, and these cells lacking MUC1 expression are not labelled. B16-muc1 (B16FO transfected with Muc1) however, are labelled.

### Key to Figure 7:

Sec only = second antibody only control to give background fluorescence levels.
Non imm = control sera from unimmunised animals
hepB cont = control sera from animals inmmunised with empty vector (no MUC1 DNA)
hepB2TR mut4 & hepB2TR mut5 are sera from animals immunised with these mutant MUC1 DNA's.

These data confirm the utility of these variant VNTR sequences in nucleic acid vaccination.

### SEQUENCE LISTING

<110> Glaxo Group Limited
   Ellis, Jonathan H
   Crowe, James S
<120> Improvements in Nucleic Acid Vaccination
<130> PG3886
<140> PCT/GBOO/04906
   <141> 2000-12-20
<150> GB 9930359.6
   <151> 1999-12-22
<160> 50
<170> PatentIn version 3.0
<210> 1
   <211> 20
   <212> PRT
   <213> Homo sapiens
<400> 1
   Pro Ala His Gly Val Thr Ser Ala Pro Asp Thr Arg Pro Ala Pro Gly
   1               5                   10                  15
   Ser Thr Ala Pro
               20
<210> 2
   <211> 20
   <212> PRT
   <213> Artificial
<220>
   <223> Mut51
<400> 2
   Pro Ala His Gly Val Val Ala Ala Pro Asp Ile Arg Pro Ala Pro Gly
   1               5                   10                  15
   Ala Val Ala Pro
               20
<210> 3
   <211> 60
   <212> DNA
   <213> Artificial
<220>
   <223> Mut51
<400> 3c
   cggcgcatg gtgtcgtagc tgccccagac atccggcccg caccgggtgc ggttgcccct 60
<210> 4
   <211> 20
   <212> PRT
   <213> Artificial
<220>
   <223> Mut5V
<400> 4
   Pro Ala His Gly Val Val Ala Ala Pro Asp Val Arg Pro Ala Pro Gly
   1 5 10 15
   Ala Val Ala Pro
   20
<210> 5
   <211> 60
   <212> DNA
   <213> Artificial
<220>
   <223> Mut 5V
<400> 5
   ccggcgcatg gtgtcgtagc tgccccagac gtggggcccg caccgggtgc ggttgcccct 60
<210> 6
   <211> 40
   <212> PRT
   <213> Artificial
<220>
   <223> Synthetic peptide
<400> 6
   Pro Ala His Gly Val Thr Ser Ala Pro Asp Thr Arg Pro Ala Pro Gly
   1 5 10 15
   Ser Thr Ala Pro Pro Ala His Gly Val Thr Ser Ala Pro Asp Thr Arg
   20 25 30
   Pro Ala Pro Gly Ser Thr Ala Pro
   35 40
<210> 7
   <211> 40
   <212> PRT
   <213> Artificial
<220>
   <223> Synthetic peptide
<400> 7
   Pro Ala His Gly Val Val Ala Ala Pro Asp Thr Arg Pro Ala Pro Gly
   1 5 10 15
   Ala Val Ala Pro Pro Ala His Gly Val Val Ala Ala Pro Asp Thr Arg
   20 25 30
   Pro Ala Pro Gly Ala Val Ala Pro
   35 40
<210> 8
   <211> 40
   <212> PRT
   <213> Artificial
<220>
   <223> Synthetic peptide
<400> 8
   Pro Ala His Gly Val Val Ala Ala Pro Asp Ala Arg Pro Ala Pro Gly
   1 5 10 15
   Ala Val Ala Pro Pro Ala His Gly Val Val Ala Ala Pro Asp Ala Arg
   20 25 30
   Pro Ala Pro Gly Ala Val Ala Pro
   35 40
<210> 9
   <211> 40
   <212> PRT
   <213> Artificial
<220>
   <223> Synthetic peptide
<400> 9
   Pro Ala His Gly Val Val Ala Ala Pro Asp Ile Arg Pro Ala Pro Gly
   1 5 10 15
   Ala Val Ala Pro Pro Ala His Gly Val Val Ala Ala Pro Asp Ile Arg
   20 25 30
   Pro Ala Pro Gly Ala Val Ala Pro
   35 40
<210> 10
   <211> 40
   <212> PRT
   <213> Artificial
<220>
   <223> Synthetic peptide
<400> 10
   Pro Ala His Gly Val Val Ala Ala Pro Asp Asn Arg Pro Ala Pro Gly
   1 5 10 15
   Ala Val Ala Pro Pro Ala His Gly Val Val Ala Ala Pro Asp Asn Arg
   20 25 30
   Pro Ala Pro Gly Ala Val Ala Pro
   35 40
<210> 11
   <211> 40
   <212> PRT
   <213> Artificial
<220>
   <223> Synthetic peptide
<400> 11
   Pro Ala His Gly Val Val Ala Ala Pro Asp Pro Arg Pro Ala Pro Gly
   1 5 10 15
   Ala Val Ala Pro Pro Ala His Gly Val Val Ala Ala Pro Asp Pro Arg
   20 25 30
   Pro Ala Pro Gly Ala Val Ala Pro
   35 40
<210> 12
   <211> 40
   <212> PRT
   <213> Artificial
<220>
   <223> Synthetic peptide
<400> 12
   Pro Ala His Gly Val Val Ala Ala Pro Asp Val Arg Pro Ala Pro Gly
   1 5 10 15
   Ala Val Ala Pro Pro Ala His Gly Val Val Ala Ala Pro Asp Val Arg
   20 25 30
   Pro Ala Pro Gly Ala Val Ala Pro
   35 40
<210> 13
   <211> 40
   <212> PRT
   <213> Artificial
<220>
   <223> Synthetic peptide
<400> 13
   Pro Ala His Gly Val Val Ala Ala Pro Asp Trp Arg Pro Ala Pro Gly
   1 5 10 15
   Ala Val Ala Pro Pro Ala His Gly Val Val Ala Ala Pro Asp Trp Arg
   20 25 30
   Pro Ala Pro Gly Ala Val Ala Pro
   35 40
<210> 14
   <211> 43
   <212> DNA
   <213> Artificial
<220>
   <223> Primer
<400> 14
   atcctacgga ccgtacagtt actcagcaca cacagcacct cac 43
<210> 15
   <211> 43
   <212> DNA
   <213> Artificial
<220>
   <223> Primer
<400> 15
   ccgctcgagc gccggcgatc ttggacctgg gagtggacac ctg 43
<210> 16
   <211> 65
   <212> DNA
   <213> Artificial
<220> ,
   <223> Primer
<400> 16
   atcctacgcc ggcgggaccc ggacctatga aaaacttaga ctgttgggtc gacaacgaag 60
   aagac 65
<210> 17
   <211> 39
   <212> DNA
   <213> Artificial
<220>
   <223> Primer
<400> 17
   gggctcgagt tagtcgttgg tccaaccttc atcggtcgg 39
<210> 18
   <211> 41
   <212> DNA
   <213> Artificial
<220>
   <223> Primer
<400> 18
   gatgtggtcg acgacattga cccttataaa gaatttggag c 41
<210> 19
   <211> 47
   <212> DNA
   <213> Artificial
<220>
   <223> Primer
<400> 19
   gtagagctcg agctaacatt gagattcccg agattgagat cttctgc 47
<210> 20
   <211> 24
   <212> DNA
   <213> Artificial
<220>
   <223> Primer
<400> 20
   ggctgcgcgt tccgaaagtt tctg 24
<210> 21
   <211> 24
   <212> DNA
   <213> Artificial
<220>
   <223> Primer
<400> 21
   ccgtgaggac aacaacatca ctct 24
<210> 22
   <211> 24
   <212> DNA
   <213> Artificial
<220>
   <223> Primer
<400> 22
   ctactaccga cgtctgtaca acgg 24
<210> 23
   <211> 24
   <212> DNA
   <213> Artificial
<220>
   <223> Primer
<400> 23
   gtttcttcat agagctgatg atgg 24
<210> 24
   <211> 23
   <212> DNA
   <213> Artificial
<220>
   <223> Primer
<400> 24
   gaagatacgg aacttgtcga tgg 23
<210> 25
   <211> 25
   <212> DNA
   <213> Artificial
<220>
   <223> Primer
<400> 25
   gtaagaaacg tacagtttga tgaag 25
<210> 26
   <211> 36
   <212> DNA
   <213> Artificial
<220>
   <223> Primer
<400> 26
   gattgtgcta gcccagccca cggagttgtt gctgcc 36
<210> 27
   <211> 51
   <212> DNA
   <213> Artificial
<220>
   <223> Primer
<400> 27
   tcgcgtatct ggcgctgcga cgacaccatg tgctggaggg gccactgctc c 51
<210> 28
   <211> 54
   <212> DNA
   <213> Artificial
<220>
   <223> Primer
<400> 28
   gattgtgcta gcaggtgcta cggcgccggg agccggtcgc gtatctggcg ctgc 54
<210> 29
   <211> 30
   <212> DNA
   <213> Artificial
<220>
   <223> Primer
<400> 29
   gattgtgcta gcccggcgca tggtgtcgtc 30
<210> 30
   <211> 27
   <212> DNA
   <213> Artificial
<220>
   <223> Primer
<400> 30
   gattgtgcta gcaggggcca ctgctcc 27
<210> 31
   <211> 86
   <212> DNA
   <213> Artificial
<220>
   <223> Oligonucleotide
<400> 31
   atcctacgcc ggcgcatggt gtcgtcgccg ccccggacac caggccggcc ccgggcgctg 60
   ttgccccccc cgctcacgga gttgtg 86
<210> 32
   <211> 92
   <212> DNA
   <213> Artificial
<220>
   <223> Oligonucleotide
<400> 32
   agcggccaca actccgtgag cggggggggc aacagcgccc ggggccggcc tggtgtccgg 60
   ggcggcgacg acaccatgcg ccggcgtagg at 92
<210> 33
   <211> 144
   <212> DNA
   <213> Artificial
<220>
   <223> Oligonucleotide
<400> 33
   gccgctcccg atactcgacc tgcacccgga gctgtcgctc cacctgcaca cggcgtggtt 60
   gcagcacctg acacacgtcc ggcgccaggc gcggtggcgc ccccagccca cggggtagta 120
   gccgcgccgg acacgcgccc cgcc 144
<210> 34
   <211> 132
   <212> DNA
   <213> Artificial
<220>
   <223> Oligonucleotide
<400> 34
   gcgcgtgtcc ggcgcggcta ctaccccatg ggctgggggc gccaccgcgc ctggcgccgg 60
   acgtgtgtca ggtgctgcaa ccacgccgtg tgcaggtgga gcgacagctc cgggtgcagg 120
   tcgagtatcg gg 132
<210> 35
   <211> 145
   <212> DNA
   <213> Artificial
<220>
   <223> Oligonucleotide
<400> 35
   ccgggagccg tcgccccccc agcccacgga gttgttgctg cccccgacac ccggcccgca 60
   ccgggagcag tggcccctcc tgcaggaccc ggacctatga aaaacttaga ctgttgggtc 120
   gacgtcggcc atagcggccg cggaa 145
<210> 36
   <211> 151
   <212> DNA
   <213> Artificial
<220>
   <223> Oligonucleotide
<400> 36
   ttccgcggcc gctatggccg acgtcgaccc aacagtctaa gtttttcata ggtccgggtc 60
   ctgcaggagg ggccactgct cccggtgcgg gccgggtgtc gggggcagca acaactccgt 120
   gggctggggg ggcgacggct cccggggcgg g 151
<210> 37
   <211> 86
   <212> DNA
   <213> Artificial
<220>
   <223> Oligonucleotide
<400> 37
   atcctacgcc ggcgcatggt gtcgtcgccg ccccggacaa taggccggcc ccgggcgctg 60
   ttgccccccc cgctcacgga gttgtg 86
<210> 38
   <211> 92
   <212> DNA
   <213> Artificial
<220>
   <223> Oligonucleotide
<400> 38
   agcggccaca actccgtgag cggggggggc aacagcgccc ggggccggcc tattgtccgg 60
   ggcggcgacg acaccatgcg ccggcgtagg at 92
<210> 39
   <211> 144
   <212> DNA
   <213> Artificial
<220>
   <223> Oligonucleotide
<400> 39
   gccgctcccg ataatcgacc tgcacccgga gctgtcgctc cacctgcaca cggcgtggtt 60
   gcagcacctg acaaccgtcc ggcgccaggc gcggtggcgc ccccagccca cggggtagta 120
   gccgcgccgg acaatcgccc cgcc 144
<210> 40
   <211> 132
   <212> DNA
   <213> Artificial
<220>
   <223> oligonucleotide
<400> 40
   gcgattgtcc ggcgcggcta ctaccccatg ggctgggggc gccaccgcgc ctggcgccgg 60
   acggttgtca ggtgctgcaa ccacgccgtg tgcaggtgga gcgacagctc cgggtgcagg 120
   tcgattatcg gg 132
<210> 41
   <211> 145
   <212> DNA
   <213> Artificial
<220>
   <223> Oligonucleotide
<400> 41
   ccgggagccg tcgccccccc agcccacgga gttgttgctg cccccgacaa tcggcccgca 60
   ccgggagcag tggcccctcc tgcaggaccc ggacctatga aaaacttaga ctgttgggtc 120
   gacgtcggcc atagcggccg cggaa 145
<210> 42
   <211> 151
   <212> DNA
   <213> Artificial
<220>
   <223> Oligonucleotide
<400> 42
   ttccgcggcc gctatggccg acgtcgaccc aacagtctaa gtttttcata ggtccgggtc 60
   ctgcaggagg ggccactgct cccggtgcgg gccgattgtc gggggcagca acaactccgt 120
   gggctggggg ggcgacggct cccggggcgg g 151
<210> 43
   <211> 20
   <212> PRT
   <213> Artificial
<220>
   <223> Mut4
<400> 43
   Pro Ala His Gly Val Val Ala Ala Pro Asp Thr Arg Pro Ala Pro Gly
   1 5 10 15
   Ala Val Ala Pro
   20
<210> 44
   <211> 20
   <212> PRT
   <213> Artificial
<220>
   <223> Mut5N
<400> 44
   Pro Ala His Gly Val Val Ala Ala Pro Asp Asn Arg Pro Ala Pro Gly
   1               5                   10                  15
   Ala Val Ala Pro
               20
<210> 45
   <211> 170
   <212> DNA
<213> Artificial
<220>
   <223> Plasmid
<220>
   <221> CDS
   <222> (42)..(158)
<400> 45
   ctagccggac cgtacagtta ctcagcacac agcacctcac c atg gga tgg agc tgt      56
                                                 Met Gly Trp Ser Cys
                                                 1               5
   atc atc ctc ttc ttg gta gca aca gct aca ggt gtc cac tcc cag gtc       104
   Ile Ile Leu Phe Leu Val Ala Thr Ala Thr Gly Val His Ser Gln Val
                   10                  15                  20
   caa aga tcg ccg gcg gga ccc gga cct atg aaa aac tta gac tgt tgg       152
   Gln Arg Ser Pro Ala Gly Pro Gly Pro Met Lys Asn Leu Asp Cys Trp
               25                  30                  35
   gtc gac aacgaagaag ac                                                 170
   Val Asp
<210> 46
   <211> 39
   <212> PRT
   <213> Artificial
<400> 46
   Met Gly Trp Ser Cys Ile Ile Leu Phe Leu Val Ala Thr Ala Thr Gly
   1 5 10 15
   Val His Ser Gln Val Gln Arg Ser Pro Ala Gly Pro Gly Pro Met Lys
   20 25 30
   Asn Leu Asp Cys Trp Val Asp
   35
<210> 47
   <211> 18
   <212> DNA
   <213> Artificial
<220>
   <223> Vaccine construct
<220>
   <221> misc feature
   <222> 3, 6, 12, 15, 18
   <223> n may be the nucleotide a, g, c or t/u
<400> 47
   gcnccngayg tncgnccn 18
<210> 48
   <211> 18
   <212> RNA
   <213> Artificial
<220>
   <223> Vaccine construct
<220>
   <221> misc feature
   <222> 3, 6, 12, 15, 18
   <223> n may be the nucleotide a, g, c or t/u
<400> 48
   gcnccngayg uncgnccn 18
<210> 49
   <211> 18
   <212> DNA
   <213> Artificial
<220>
   <223> Vaccine construct
<220>
   <221> misc_feature
   <222> 3, 6, 15, 18
   <223> n may be the nucleotide a, g, c or t/u
<400> 49
   gcnccngaya thcgnccn 18
<210> 50
   <211> 18
   <212> RNA
   <213> Artificial
<220>
   <223> Vaccine construct
<220>
   <221> misc_feature
   <222> 3, 6, 15, 18
   <223> n may be the nucleotide a, g, c or t/u
<400> 50 gcnccngaya uhcgnccn 18

## Claims

1. A nucleic acid vaccine construct comprising an isolated polynucleotide which encodes a polypeptide comprising an altered MUC-1 VNTR monomer, in which the construct comprises the sequence shown in Figure 2 or Figure 3, and in which the encoded polypeptide, when expressed, can bind the antibody SM3.

2. A nucleic acid vaccine construct according to claim 1, which further comprises a further polynucleotide sequence encoding a heterologous polypeptide.

3. A nucleic acid vaccine construct according to claim 2 wherein the heterologous polypeptide is tetanus toxin, hepatitis B virus core protein, hepatitis B virus surface protein, ovalbumin, glutathione S trasferase, keyhole limpet haemacyanin, or pigeon cytochrome C.

4. A vaccine composition comprising a nucleic acid according to any preceding claim in combination with a pharmaceutically acceptable carrier.

5. A nucleic acid vaccine construct according to any of claims 1 to 3, or a composition according to claim 4 for use in vaccination of a mammal against tumours.

6. Use of a nucleic acid vaccine construct according to any of claims 1 to 3 or a composition according to claim 4 in the manufacture of a medicament for use in vaccination of a mammal against tumours.

7. Use according to claim 6 wherein the construct or composition is administered using a gene gun.

8. Use according to claim 6 or 7 wherein the tumours are epithelial cell tumours.

9. Use according to claim 8 wherein the tumours are breast cancer tumours.

## Patentansprüche

1. Nukleinsäure-Vakzinkonstrukt, umfassend ein isoliertes Polynukleotid, das ein Polypeptid codiert, umfassend ein verändertes MUC-1 VNTR-Monomer, worin das Konstrukt die in Figur 2 oder Figur 3 dargestellte Sequenz umfaßt und worin das codierte Polypeptid, wenn es exprimiert wird, an den Antikörper SM3 binden kann.

2. Nukleinsäure-Vakzinkonstrukt gemäß Anspruch 1, das ferner eine weitere Polynukleotidsequenz umfaßt, codierend ein heterologes Polypeptid.

3. Nukleinsäure-Vakzinkonstrukt gemäß Anspruch 2, wobei das heterologe Polypeptid Tetanustoxin, Hepatitis B-Virus-Kernprotein, Hepatitis B-Virus-Oberflächenprotein, Ovalbumin, Gluthathion S-Transferase, Schlüsselloch-Schneckenhämocyanin oder Tauben-Cytochrom-C ist.

4. Vakzinzusammensetzung, umfassend eine Nukleinsäure gemäß einem der vorstehenden Ansprüche in Kombination mit einem pharmazeutisch annehmbaren Träger.

5. Nukleinsäure-Vakzinkonstrukt gemäß einem der Ansprüche 1 bis 3 oder eine Zusammensetzung gemäß Anspruch 4 zur Verwendung bei der Impfung eines Säugers gegen Tumoren.

6. Verwendung eines Nukleinsäure-Vakzinkonstrukts gemäß einem der Ansprüche 1 bis 3 oder einer Zusammensetzung gemäß Anspruch 4 bei der Herstellung eines Medikaments zur Verwendung bei der Impfung eines Säugers gegen Tumoren.

7. Verwendung gemäß Anspruch 6, wobei das Konstrukt oder die Zusammensetzung unter Verwendung einer Genpistole verabreicht wird.

8. Verwendung gemäß Anspruch 6 oder 7, wobei die Tumoren Epithelzelltumoren sind.

9. Verwendung gemäß Anspruch 8, wobei die Tumoren Brustkrebstumoren sind.

## Revendications

1. Construction vaccinale à base d'acide nucléique comprenant un polynucléotide isolé qui code pour un polypeptide comprenant un monomère MUC-1 VNTR modifié, dans laquelle la construction comprend la séquence présentée sur la figure 2 ou la figure 3, et dans laquelle le polypeptide codé, lorsqu'il est exprimé, peut se lier à l'anticorps SM3.

2. Construction vaccinale à base d'acide nucléique selon la revendication 1, qui comprend en outre une autre séquence de polynucléotides codant pour un polypeptide hétérologue.

3. Construction vaccinale à base d'acide nucléique selon la revendication 2, dans laquelle le polypeptide hétérologue est la toxine tétanique, la protéine de capside du virus de l'hépatite B, la protéine de surface du virus de l'hépatite B, l'ovalbumine, la glutathione S-transférase, l'hémocyanine de patelle, ou le cytochrome C de pigeon.

4. Composition vaccinale comprenant un acide nucléique selon l'une quelconque des revendications précédentes, en combinaison avec un véhicule pharmaceutiquement acceptable.

5. Construction vaccinale à base d'acide nucléique selon l'une quelconque des revendications 1 à 3, ou composition selon la revendication 4, destinée à être utilisée dans la vaccination d'un mammifère contre les tumeurs.

6. Utilisation d'une construction vaccinale à base d'acide nucléique selon l'une quelconque des revendications 1 à 3, ou d'une composition selon la revendication 4, dans la fabrication d'un médicament destiné à être utilisé dans la vaccination d'un mammifère contre les tumeurs.

7. Utilisation selon la revendication 6, dans laquelle la construction ou la composition est administrée en utilisant un canon à gènes.

8. Utilisation selon la revendication 6 ou 7, dans laquelle les tumeurs sont des tumeurs des cellules épithéliales.

9. Utilisation selon la revendication 8, dans laquelle les tumeurs sont des tumeurs cancéreuses du sein.
